(19) **Europäisches Patentamt / European Patent Office / Office européen des brevets**

(11) **EP 4 715 039 A1**

(12) **EUROPEAN PATENT APPLICATION**
published in accordance with Art. 153(4) EPC

(43) Date of publication:
**25.03.2026 Bulletin 2026/13**

(21) Application number: **24810504.1**

(22) Date of filing: **20.06.2024**

(51) International Patent Classification (IPC):
**C12N 1/16** *(2026.01)* **C12N 1/02** *(2006.01)*
**A23C 9/12** *(2006.01)* **C12R 1/645** *(2006.01)*

(52) Cooperative Patent Classification (CPC):
**A21C 13/00; A23C 9/12; A23L 7/104; C12G 3/02; C12N 1/02; C12N 1/16;** C12R 2001/645

(86) International application number:
**PCT/CN2024/100392**

(87) International publication number:
**WO 2024/240269 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.05.2023 CN 202310574232**

(71) Applicant: **Angel Yeast Co., Ltd**
**Yichang, Hubei 443003 (CN)**

(72) Inventors:
• **SUN, Yafang**
**Yichang, Hubei 443003 (CN)**
• **QIN, Xianwu**
**Yichang, Hubei 443003 (CN)**

• **HUANG, Yueyan**
**Yichang, Hubei 443003 (CN)**
• **ZHANG, Yan**
**Yichang, Hubei 443003 (CN)**
• **GUO, Tianfen**
**Yichang, Hubei 443003 (CN)**
• **LI, Jing**
**Yichang, Hubei 443003 (CN)**

(74) Representative: **karo IP**
**Patentanwälte PartG mbB**
**Steinstraße 16-18**
**40212 Düsseldorf (DE)**

Remarks:
The complete document including Reference Table(s) and the Sequence Listing(s) can be downloaded from the EPO website

(54) **KLUYVEROMYCES MARXIANUS STRAIN AND USE THEREOF**

(57)    A *Kluyveromyces marxianus* strain and a use thereof. The *Kluyveromyces marxianus* strain is *Kluyveromyces marxianus* AMCC30648, which is deposited in the China Center for Type Culture Collection (CCTCC), and the deposit number is CCTCC NO: M 20222108; or *Kluyveromyces marxianus* AMCC31343, which is deposited in the China Center for Type Culture Collection (CCTCC) and the deposit number is CCTCC NO: M 20222111. The *Kluyveromyces marxianus* strain can generate a plurality of fragrance substances which are high in concentration. The strain can be applied to the application fields such as brewing, beverages, and baking.

EP 4 715 039 A1

## Description

## Technical Field

The present invention belongs to the field of microorganisms, and more particularly, relates to a *Kluyveromyces marxianus* strain and a use thereof.

## Background Art

*Kluyveromyces marxianus* is one of the non-traditional yeasts, which is usually isolated from fermented traditional dairy products, beer wort and grapes. *Kluyveromyces marxianus* has enzyme production capacity, high temperature resistance, good safety, and a wide range of substrate utilization and can produce fragrant compounds such as ethyl acetate to endow the product with a unique fragrance, and thus is a class of microorganisms that are widely used in the food industry and are recognized as harmless to human being..

## Summary of the Invention

Fragrance substances produced by *Kluyveromyces marxianus* in the prior art lack diversity or are relatively low in content, and cannot meet the requirements.

In view of the problems in the prior art, the present invention provides a *Kluyveromyces marxianus* strain capable of producing a variety of fragrance substances with high content.

In a first aspect, the present invention provides a *Kluyveromyces marxianus* strain.

The *Kluyveromyces marxianus* strain is *Kluyveromyces marxianus* AMCC30648, which is deposited in the China Center for Type Culture Collection (CCTCC), and the deposit number is CCTCC NO: M 20222108; or *Kluyveromyces marxianus* AMCC31343, which is deposited in the China Center for Type Culture Collection (CCTCC), and the deposit number is CCTCC NO: M 20222111.

Preferably, an ITS gene sequence of the *Kluyveromyces marxianus* AMCC30648 is as shown in SEQ ID NO: 1.

Preferably, an ITS gene sequence of the *Kluyveromyces marxianus* AMCC31343 is as shown in SEQ ID NO: 2.

In a second aspect, the present invention provides a fermented preparation method of a *Kluyveromyces marxianus* microbial agent. The method includes the following step: culturing the *Kluyveromyces marxianus* strain.

Preferably, the preparation method further includes the following steps:

(1) performing amplified culture on the *Kluyveromyces marxianus* strain; and
(2) adding the product obtained in step (1) to a medium and performing fermented culture at 26-42°C;

preferably, the medium being a liquid medium or a solid medium.

In a third aspect, the present invention provides a microbial agent, the microbial agent containing the *Kluyveromyces marxianus* strain.

Preferably, the microbial agent is obtained by the fermented preparation method.

In a fourth aspect, the present invention provides a use of the *Kluyveromyces marxianus* strain or the microbial agent in the fermentation.

In a fifth aspect, the present invention provides a use of the *Kluyveromyces marxianus* strain or the microbial agent in the fermentation of dairy products, fermented production of liquor or fermentation of flour products.

In a sixth aspect, the present invention provides a flour dough, the flour dough containing the *Kluyveromyces marxianus* strain or the microbial agent.

Preferably, the flour dough contains one or a combination of two or more of acetoin, phenethanol, isoamyl acetate, phenethyl acetate, ethyl acetate, ethyl caprylate, and phenethyl isobutyrate.

In a seventh aspect, the present invention provides a flour paste, the flour paste containing the *Kluyveromyces marxianus* strain or the microbial agent.

Preferably, the flour paste contains one or a combination of two or more of acetoin, phenethanol, isoamyl acetate, phenethyl acetate, ethyl acetate, ethyl caprylate, and phenethyl isobutyrate.

Preferably, a mass ratio of the *Kluyveromyces marxianus* strain to flour to water in the flour paste is (0.1-5): (200-500): (500-1000).

In an eighth aspect, the present invention provides a flour product, a fermentation microbial agent for the flour product containing the *Kluyveromyces marxianus* strain or the microbial agent.

Preferably, the flour product contains one or a combination of two or more of acetoin, phenethanol, and 2,3-butanedione.

In a ninth aspect, the present invention provides a fermentation broth, a fermentation microbial agent for the fermentation broth containing the *Kluyveromyces marxianus* strain or the microbial agent.

Preferably, the fermentation broth contains phenethyl acetate, phenethanol, ethyl acetate, isoamyl acetate, farnesol, phenethyl isobutyrate, and ethyl caprylate.

In a tenth aspect, the present invention provides a liquor, a fermentation microbial agent for the liquor containing the *Kluyveromyces marxianus* strain or the microbial agent.

The *Kluyveromyces marxianus* strain provided by the present invention can produce a plurality of fragrance substances which are high in concentration. The strain can be applied to the application fields such as brewing, beverages, and baking. Pasta prepared by the strain provided by the present invention can produce acetoin with butter fragrance, as well as phenethanol, phenethyl acetate and the like with fruity fragrance, etc.

## Strain preservation information

The *Kluyveromyces marxianus* AMCC30648 in the present invention is deposited in the China Center for Type Culture Collection (CCTCC) on December 30, 2022, the deposit number is CCTCC NO: M 20222108, the preservation address is Wuhan University, Wuhan, China, the postal code is 430072, and the telephone is 027-68754052.

The *Kluyveromyces marxianus* AMCC31343 provided by the present invention is deposited in the China Center for Type Culture Collection (CCTCC) on December 30, 2022, the deposit number is CCTCC NO: M 20222111, the preservation address is Wuhan University, Wuhan, China, the postal code is 430072, and the telephone is 027-68754052.

## Brief Description of the Drawings

FIG. 1 shows a colony morphology picture of *Kluyveromyces marxianus* AMCC30648;

FIG. 2 shows a microexamination picture of the *Kluyveromyces marxianus* AMCC30648;

FIG. 3 shows a colony morphology picture of *Kluyveromyces marxianus* AMCC31343;

FIG. 4 shows a microexamination picture of the *Kluyveromyces marxianus* AMCC31343;

FIG. 5 shows an analysis and comparison chart of the *Kluyveromyces marxianus* AMCC31343 and a commercially available strain in terms of the content of fragrance substances in a fermented flour paste, wherein a is a comparison chart of the acetoin content, and b is a comparison chart of the phenethanol content; and

FIG. 6 shows an analysis and comparison chart of the *Kluyveromyces marxianus* AMCC31343 and a commercially available strain in terms of the content of fragrance substances in a fermented steamed bun, wherein a is a comparison chart of the acetoin content, and b is a comparison chart of the phenethanol content.

## Detailed Description of the Invention

A strain provided by the present invention is a strain obtained by ARTP mutation screening of fragrance-producing *Kluyveromyces marxianus* AMCC30648 that is obtained by isolation and identification. Compared with a starting strain, among fragrance substances produced by *Kluyveromyces marxianus* AMCC31343 provided by the present invention, the phenethyl acetate content is increased by 15.9%, the phenethanol content is increased by 25.9%, and the phenethanol content is increased by 144.6%. The *Kluyveromyces marxianus* AMCC31343 provided by the present invention may be applied to a variety of fields, such as fragrant bread making, fragrant yogurt fermentation, etc., achieving a significant application effect.

The components of a medium involved in the following examples were as follows:

YPD medium: 10 g of yeast extract powder, 20 g of glucose, 20 g of peptone, 20 g of agar, and 1000 mL of water, all of which were sterilized at 115°C for 30 min.

Source information of reagents and instruments involved in the examples of the present invention are shown in Table 1 and Table 2.

Table 1 Reagent information table

| Reagent | Manufacturer |
| --- | --- |
| Yeast extract powder | Angel Yeast Co., Ltd. |
| Glucose | Sinopharm Chemical Reagents Co., Ltd. (Hushi®) |
| Peptone | Angel Yeast Co., Ltd. |
| Agar | Shanghai Huixing Bio-Chem Reagents Co., Ltd. |
| 2×PCR Mix | TianGen Biotech (Beijing) Co., Ltd. |

Table 2 Instrument information table

| Instrument | Model | Manufacturer |
|---|---|---|
| Analytical balance | ME4002E | METTLER TOLEDO |
| pH meter | PB-10 | Sartorius |
| Superclean bench | SKJH-1109 | Shanghai Sukun Industrial Co., Ltd. |
| Constant temperature shaker | ZWYR-2102C | Shanghai Zhicheng Analytical Instrument Manufacturing Co., Ltd. |
| Biochemical incubator | SPX-158L | Ningbo Xinzhi Biotechnology Co., Ltd. |
| Thermostat water bath | HH-2 | Guohua (Changzhou, Jiangsu) Instrument Manufacturing Co., Ltd. |
| Centrifuge | DL-5200B-II | Shanghai Anting Scientific Instrument Factory(Feige®) |
| PCR Instrument | C1000 | Bole Life Medical Products (Shanghai) Co., Ltd.(BIO-RAD) |
| Gel imaging system | GelDocTMXR+ | Bole Life Medical Products (Shanghai) Co., Ltd.(BIO-RAD) |
| Optical microscope | CX43 | OLYMPUS |
| Fully automatic growth curve analyzer | BioscreenC | OY Growth Curves |
| Gas chromatography-mass spectrometer | 7890B-5977B | Agilent Technologies, Inc.(Agilent GC-MS) |

## Example 1: Obtaining of *Kluyveromyces marxianus* AMCC31343

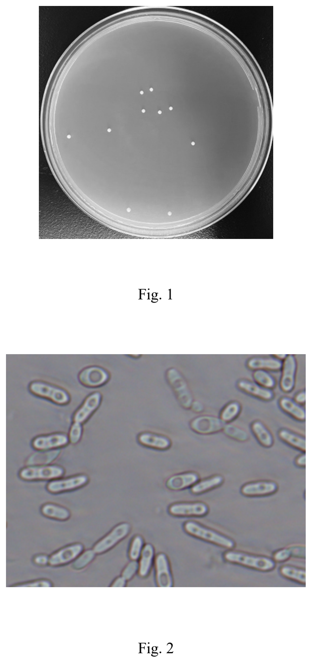

Fermented dairy product from Shannan City, Xizang Autonomous Region, China was dissolved in sterile water and mixed well; a yeast suspension was pipetted and diluted in 10-fold series to prepare a $10^{-5}$ yeast suspension and a $10^{-6}$ yeast suspension, which were then coated in a YPD medium and cultured at 30°C for 40 h; the morphology of the yeast in a prepared slide was observed under a microscope, while the characteristics of single colonies on a flat plate were observed; and a strain with typical yeast colony characteristics was isolated, subjected to streak purification, inoculated in a YPD inclined medium, and stored at 4°C. It was observed that colonies of one strain were cheese-like in character, milky white in color, and smooth in surfaces; and oval and slender in micromorphology, and presented budding and reproductive under microexamination. A genome of the yeast strain was extracted. Taking ITS5(5'-GGAAGTAAAAGTCGTAACAAGG-3) (a serial number of SEQ ID NO: 3), and ITS4(5'-TCCTCCGCTTATTGATATGC-3') (a serial number of SEQ ID NO: 4) as primers, a PCR procedure was pre-denaturation at 94°C for 5 min, denaturation at 94°C for 30 s, annealing at 55°C for 45 s, extension at 72°C for 90 s, in a total of 30 cycles, and finally extension at 72°C for 10 min for internal transcribed spacer (ITS) sequence amplification of the yeast. After 1% gel electrophoresis detection and sequencing, the sequences were compared with those on GenBank using Blast analysis, and the sequences with a similarity greater than 99% were considered to be from the same species. In conjunction with morphological analysis and molecular identification, this strain was *Kluyveromyces marxianus,* and this strain was named *Kluyveromyces marxianus* AMCC30648. FIG. 1 showed a colony morphology picture of *Kluyveromyces marxianus* AMCC30648. FIG. 2 showed a microexamination picture of the *Kluyveromyces marxianus* AMCC30648. the *Kluyveromyces marxianus* AMCC30648 was deposited in the China Center for Type Culture Collection (CCTCC) on December 30, 2022, and the deposit number was CCTCC NO: M 20222108.

An ITS gene sequence of the *Kluyveromyces marxianus* AMCC30648 was as shown in SEQ ID NO: 1:

TCTTCCCCCCCCTTTTTGTATGCTTAAGTTCAGCGGGTACTCCTACCTGATTTGAGGTC

AAACTTTGAGAGTTTTGGTTAAAGCCGTATGCCTCAAGGAGACAAACACCAGCGAGTC

TTTATAACACCTATGAGTCTCTATGACCCAAGCTTACCACGAATTGGCGCAAACCTAAG

ACGTAGATGTGCAAGAGTCGAGTCCATAGACTTGACACGCAGCCCTGCTCACGCAGAT

GGCAACGGCTAGCCACTTTCAAGTTAACCCGAGACGAGTATCACTCACTACCAAACCC

AAAGGTTTGAGAGAGAAATGACGCTCAAACAGGCATGCCCCCTGGAATACCAGAGGG

CGCAATGTGCGTTCAAAGATTTGATGATTCACGAAAATCTGCAATTCACAATACATATC

GCAATTCGCTGCGTTCTTCATCGATGCGAGAACCAAGAGATCCGTTGTTGAAAGTTTTG

AATATTAAATTTTATAGTATAATAGTTTTTCATAATACAAAATATTGTTTGTGTTTATGTCC

ACTGGAGAGACGAGCTCTCCAGGGAAGTAGTTCATAGAGAAAAAACTCCATTGTGTTT

AGGATGAGAAATAGAAAACTGATAGCAGAGAATCAAGAACTGGCCGCGCAATTAAGC

GCAGGCCTTGTTCAGACGATTCCCCCAGTAATCTATTCATTCATAATCTTTAATGATCCTT

CCGCAGGTTCACCTACGGAAACCTTGTATTTTTTTATTCTCTCTATAAA.

Atmospheric room temperature plasma (ARTP) mutagenesis was performed on the *Kluyveromyces marxianus* AMCC30648. The mutagenesis conditions were as follows: a treatment distance was 2 mm; treatment power was 120 W, a gas flow rate was 10 SLM, a dispensing volume was 10 μL, and the treatment time was 0 s, 30 s, 60 s, 90 s, 120 s, 150 s, 180 s, respectively. After treatment, this strain was shaken by a vortex shaker for 1 min, diluted for YPD flat plate coating and inoculation, and cultured at 30°C for 24 h. The colonies on the flat plate were counted, and a lethality curve was plotted. Single colonies with a lethality rate of 85%-99% were respectively inoculated with YPD for liquid fermentation, and cultured at 30°C and 180 rpm for 20 h. GC-MS was used to detect supernatant fragrance substances in fermentation broth. In conjunction with biomass analysis and comparison, a mutagenic strain with a concentration of phenethyl acetate and phenethanol increased by 15% compared with the starting strain was screened. This mutagenic strain was named *Kluyveromyces marxianus* AMCC31343. Colonies of the *Kluyveromyces marxianus* AMCC31343 were uniform in character, milky white in color, and smooth in surfaces; and oval in micromorphology, and presented budding and reproductive under microexamination.

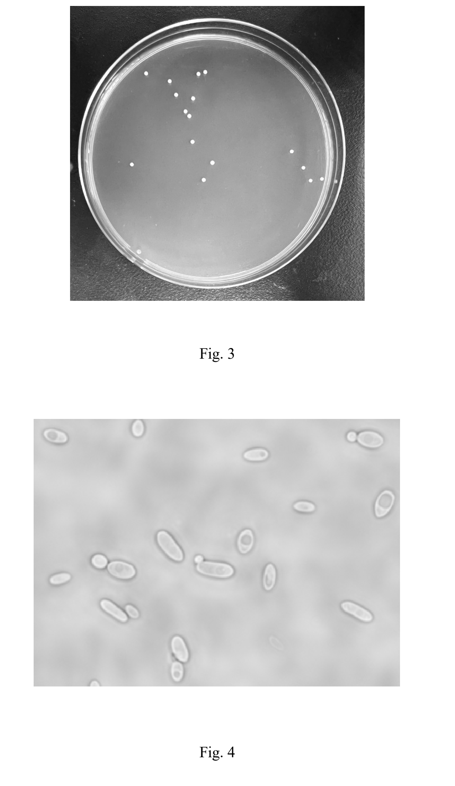

FIG. 3 showed a colony morphology picture of the *Kluyveromyces marxianus* AMCC31343. FIG. 4 showed a microexamination picture of the *Kluyveromyces marxianus* AMCC31343. The *Kluyveromyces marxianus* AMCC31343 was deposited in the China Center for Type Culture Collection (CCTCC), and the deposit number was CCTCC NO: M 20222111.

By taking ITS5(5'-GGAAGTAAAAGTCGTAACAAGG-3') (a serial number of SEQ ID NO: 3) and ITS4(5'-TC CTCCGCTTATTGATATGC-3') (a serial number of SEQ ID NO: 4) as primers, a PCR procedure was pre-denaturation at 94°C for 5 min, denaturation at 94°C for 30 s, annealing at 55°C for 45 s, extension at 72°C for 90 s, in a total of 30 cycles, and finally extension at 72°C for 10 min for internal transcribed spacer (ITS) sequence amplification of the yeast, followed by 1% gel electrophoresis detection and sequencing. An ITS sequence of the *Kluyveromyces marxianus* AMCC31343 was as shown in SEQ ID NO: 2:

CCTTCCGTAGGTGAACCTGCGGAAGGATCATTAAAGATTATGAATGAATAGATTACTGG

GGGAATCGTCTGAACAAGGCCTGCGCTTAATTGCGCGGCCAGTTCTTGATTCTCTGCTA

TCAGTTTTCTATTTCTCATCCTAAACACAATGGAGTTTTTTCTCTATGAACTACTTCCCTG

GAGAGCTCGTCTCTCCAGTGGACATAAACACAAACAATATTTTGTATTATGAAAAACTA

TTATACTATAAAATTTAATATTCAAACTTTCAACAACGGATCTCTTGGTTCTCGCATCGA

TGAAGAACGCAGCGAATTGCGATATGTATTGTGAATTGCAGATTTTCGTGAATCATCAA

ATCTTTGAACGCACATTGCGCCCTCTGGTATTCCAGGGGGCATGCCTGTTTGAGCGTCA

TTTCTCTCAAACCTTTGGGTTTGGTAGTGAGTGATACTCGTCTCGGGTTAACTTGAA

AGTGGCTAGCCGTTGCCATCTGCGTGAGCAGGGCTGCGTGTCAAGTCTATGGACTCGA

CTCTTGCACATCTACGTCTTAGGTTTGCGCCAATTCGTGGTAAGCTTGGGTCATAGAGA

CTCATAGGTGTTATAAAGACTCGCTGGTGTTTGTCTCCTTGAGGCATACGGCTTTAACC

AAAACTCTCAAAGTTTGACCTCAAATCAGGTAGGAGTACCCGCTGAACTTAAGCATAT

CAATAAGCGGAGGAA.

**Example 2: Analysis of the growth of *Kluyveromyces marxianus* AMCC31343**

(I) Analysis of growth curve: *Kluyveromyces marxianus* AMCC31343 and a starting strain, i.e., *Kluyveromyces marxianus* AMCC30648, were respectively inoculated into a test tube filled with 5 mL of YPD liquid medium, cultured at 30°C and 180 rpm for 24 h, then inoculated into a 100-well culture plate containing 300 $\mu$L of YPD liquid medium according to an inoculation amount of 3%, and prepared for on-machine determination on a Bioscreen C instrument; and parameters were set: a temperature of 30°C, a time of 24 h, and a wavelength of 600 nm; data was measured once every 30 min; a growth curve was then determined; and growth was considered to have occurred when the $OD600_{nm}$ increase rate exceeded 50%.

(II) Biomass analysis: *Kluyveromyces marxianus* AMCC31343 and a starting strain, i.e., *Kluyveromyces marxianus* AMCC30648, were respectively inoculated into a test tube filled with 5 mL of YPD liquid medium, cultured at 30°C and 180 rpm for 24 h, then inoculated into a conical flask containing 300 mL of YPD liquid medium according to an inoculation amount of 0.5%, and cultured at 30°C and 180 rpm for 24 h.

$\mu$ was a maximum specific growth rate, which was calculated according to the following formula:

$$\mu = \frac{ln OD_2 - ln OD_1}{t_2 - t_1}$$

$OD_1$-start time of the logarithmic growth period
$OD_2$-end time of the logarithmic growth period

Example 3: Comparison of the growth of *Kluyveromyces marxianus* AMCC31343 and starting strain

| Strain number | $OD_{600\text{-}24h\text{-}30°C}$ | $\mu_{30°C}$ | Biomass (g/L) |
|---|---|---|---|
| AMCC30648 | 1.695 | 0.353 | 57.5 |
| AMCC31343 | 1.707 | 0.369 | 59.7 |

The results in Table 3 showed that there was no significant difference in a maximum specific growth rate and OD value between the *Kluyveromyces marxianus* AMCC31343 and the *Kluyveromyces marxianus* AMCC30648 under a condition of 30°C, and the biomass index was increased by 3.8%.

**Example 3: Analysis of temperature tolerance of *Kluyveromyces marxianus* AMCC31343**

*Kluyveromyces marxianus* AMCC31343 and a starting strain, i.e., *Kluyveromyces marxianus* AMCC30648, were respectively inoculated into a test tube filled with 5 mL of YPD liquid medium, cultured at 30°C and 180 rpm for 24 h, then inoculated into a 100-well culture plate containing 300 $\mu$L of YPD liquid medium according to an inoculation amount of 1%, and prepared for on-machine determination on a Bioscreen C instrument; parameters were set: a temperature of 37°C, a time of 24 h, and a wavelength of 600 nm; data was measured once every 30 min; a growth curve were then determined ($\mu$ was a maximum specific growth rate, which was calculated according to the same formula as above); and a temperature tolerance test at 42°C was consistent with the above steps.

Example 4: Growth data of *Kluyveromyces marxianus* AMCC31343 and starting strain at different temperature conditions

| Strain number | $OD_{600-24h-37°C}$ | $\mu_{37°C}$ | $OD_{600-24h-42°C}$ | $\mu_{42°C}$ |
|---|---|---|---|---|
| AMCC30648 | 1.855 | 0.4823 | 1.511 | 0.2875 |
| AMCC31343 | 1.887 | 0.4892 | 1.507 | 0.2799 |

The results showed that there was no significant difference in the maximum specific growth rate and OD value between the *Kluyveromyces marxianus* AMCC31343 and the starting strain at 37°C and 42°C, both achieving high temperature resistance.

**Example 4: Analysis of volatile components in fermentation supernatant**

*Kluyveromyces marxianus* AMCC31343 and a starting strain, i.e., *Kluyveromyces marxianus* AMCC30648, were respectively inoculated into a test tube filled with 5 mL of YPD liquid medium, cultured at 30°C and 180 rpm for 20 h, then inoculated into a triangular flask containing 300 $\mu$L of YPD liquid medium according to an inoculation amount of 0.5%, cultured at 30°C and 180 rpm for 20 h, and centrifuged; a supernatant was collected, followed by 0.22 $\mu$m filtration; 10 mL of filtrate was taken, added with 20 $\mu$L of internal standard solution (n-butyl acetate), and uniformly mixed; the temperature was preserved for 2 min according to chromatographic conditions: a diversion ratio of 40: 1, a sample inlet temperature of 250°C, a detector temperature of 250°C, a chromatographic column being an AT.LZP-930 liquor column, and an initial column temperature of 50°C, and raised to 110°C at a rate of 5°C/min, and preserved for 0 min; raised to 130°C at a rate of 3°C/min, and preserved for 0 min; and raised to 230°C at a rate of 15°C/min, and preserved for 2 min; volatile components were analyzed; and the comparison of the *Kluyveromyces marxianus* AMCC31343 and the starting strain in the content of fragrance substances was shown in Table 5.

Example 5: Comparison of *Kluyveromyces marxianus* AMCC31343 and starting strain in fragrance substances determined by GC-MS

| Fragrance substances | Characteristic fragrance | CAS number | Estimated concentration (ng/g) | |
|---|---|---|---|---|
| | | | AMCC30648 | AMCC31343 |
| Phenethyl acetate | Fragrance of rose, honey, apple, and cocoa | 103-45-7 | 1857 | 2152 |
| Phenethanol | Fragrance of rose | 60-12-8 | 977.6 | 1231 |
| Ethyl acetate | Fragrance of pear and banana | 141-78-6 | 316.1 | 372.1 |
| Isoamyl acetate | Fragrance of pear and banana | 123-92-2 | 85.24 | 208.5 |
| Farnesol | Fruity fragrance and floral fragrance | 4602-84-0 | 42.53 | 69.03 |

(continued)

| Fragrance substances | Characteristic fragrance | CAS number | Estimated concentration (ng/g) | |
|---|---|---|---|---|
| | | | AMCC30648 | AMCC31343 |
| Phenethyl isobuty-rate | Fruity fragrance | 103-48-0 | 41.37 | 59.66 |
| Ethyl caprylate | Fragrance of pineapple, apple and brandy | 106-32-1 | 28.85 | 47.00 |

The results in Table 5 showed that the main fragrance substances detected in the fermentation broth were esters and alcohols, among which phenethyl acetate and phenethanol mainly presented fragrance of rose; and ethyl acetate, isoamyl acetate, farnesol, phenethyl isobutyrate and ethyl caprylate presented fruity fragrance of pear and banana. The content of isoamyl acetate in the fermentation broth of the *Kluyveromyces marxianus* AMCC31343 was increased in a maximum proportion, which was increased by 145% compared with the starting strain. The content of phenethyl acetate was the highest, which was 16% higher than that of the starting strain.

**Example 5: Analysis of fragrance components in fermented flour**

A control strain was commercially available Angel low-sugar active dry yeast (5 g/pack), and yeast used in this product was *Saccharomyces cerevisiae.*

1. Making flour paste: *Kluyveromyces marxianus* AMCC31343 and a control strain were respectively inoculated into a triangular flask containing 300 g of flour and 700 g of water at an inoculation amount of 1%, and cultured at 30°C and 200 rpm for 24 h, 48 h and 60 h, respectively.
2. Making steamed bun: 60 g of flour paste was taken, added into a triangular flask containing 300 g of flour and 105 g of water, and kneaded into a smooth flour dough, which was then put into a 100°C steamer after proofing and steamed for 15 min.

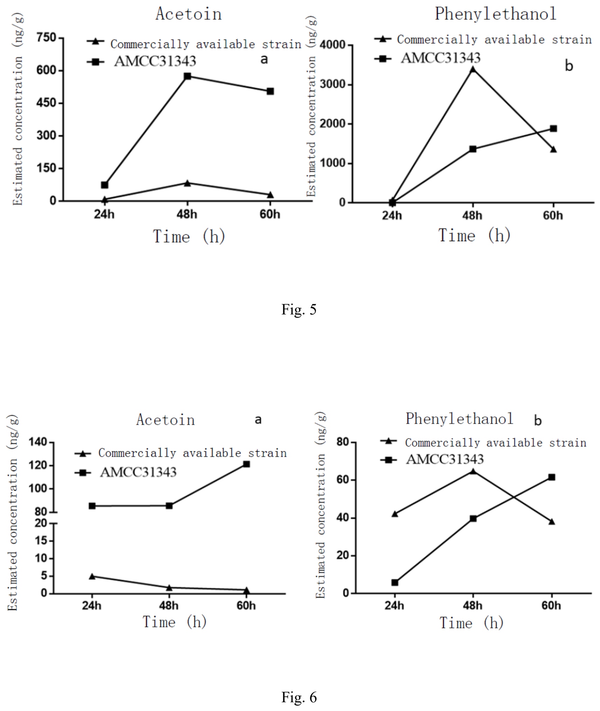

Flour paste and steamed bun samples taken at 24 h, 48 h, and 60 h were centrifuged; a supernatant was collected, followed by 0.22 $\mu$m filtration; 10 mL of filtrate was taken, added with 20 $\mu$L of internal standard solution (n-butyl acetate), and mixed well; the temperature was preserved for 2 min according to chromatographic conditions: a diversion ratio of 40: 1, a sample inlet temperature of 250°C, a detector temperature of 250°C, a chromatographic column being an AT.LZP-930 liquor column, and an initial column temperature of 50°C, raised to 110°C at a rate of 5°C/min, and preserved for 0 min; raised to 130°C at a rate of 3°C/min, and preserved for 0 min; and raised to 230°C at a rate of 15°C/min, and preserved for 2 min; and volatile components were analyzed. FIG. 5 and FIG. 6 showed the content of fragrance substances in the fermented flour paste, and FIG. 6 and FIG. 7 showed the content of fragrance substances in the fermented bun. The analysis and comparison of the fragrance components were as follows.

Example 6: Comparison of main fragrance components of *Kluyveromyces marxianus* AMCC31343 in fermented flour paste

| Fragrance substances | Characteristic fragrance | CAS number | Estimated concentration (ng/g) | | | | | |
|---|---|---|---|---|---|---|---|---|
| | | | 24 h | | 48 h | | 60 h | |
| | | | Commercially available Angel yeast | AMCC3134 3 | Commercially available Angel yeast | AMCC3134 3 | Commercially available Angel yeast | AMCC3134 3 |
| Acetoin | Butter fra-grance | 513-86-0 | 9.51 | 75.6 | 83.84 | 575.6 | 30.94 | 506.1 |
| Phenethanol | Fragrance of rose | 60-12-8 | 75.8 | 7.23 | 3403 | 1368 | 1365 | 1888 |

(continued)

| Fragrance substances | Characteristic fragrance | CAS number | Estimated concentration (ng/g) | | | | | |
|---|---|---|---|---|---|---|---|---|
| | | | 24 h | | 48 h | | 60 h | |
| | | | Commercially available Angel yeast | AMCC31343 | Commercially available Angel yeast | AMCC31343 | Commercially available Angel yeast | AMCC31343 |
| Isoamyl acetate | Fragrance of pear and banana | 123-92-2 | 1.49 | 0.61 | 137.62 | 40.32 | 176.4 | 161.4 |
| Phenethyl acetate | Fragrance of rose, honey, apple, and cocoa | 103-45-7 | - | 0.53 | 119 | 30.48 | 142.9 | 134.1 |
| Ethyl acetate | Fragrance of pear and banana | 141-78-6 | - | - | 82.67 | 17.36 | 198.5 | 940.1 |
| Ethyl caprylate | Fragrance of pineapple, apple and brandy | 106-32-1 | 1.15 | - | 64.31 | 7.085 | 12.53 | 15.24 |
| Phenethyl isobutyrate | Fruity fragrance | 103-48-0 | - | - | 5.696 | 6.069 | - | - |

Example 7: Comparison of main fragrance components of *Kluyveromyces marxianus* AMCC31343 in fermented steamed bun

| Fragrance substances | Characteristic fragrance | CAS number | Estimated concentration (ng/g) | | | | | |
|---|---|---|---|---|---|---|---|---|
| | | | 24 h | | 48 h | | 60 h | |
| | | | Commercially available Angel yeast | AMCC31343 | Commercially available Angel yeast | AMCC31343 | Commercially available Angel yeast | AMCC31343 |
| Acetoin | Butter fragrance | 513-86-0 | 4.999 | 85.52 | 1.751 | 87.81 | 1.115 | 121.5 |
| Phenethanol | Fragrance of rose | 60-12-8 | 42.32 | 5.894 | 64.84 | 39.77 | 38.26 | 61.61 |
| 2,3-butanedione | Cream fragrance | 431-03-8 | - | 7.031 | - | 2.135 | - | 1.98 |

The results in FIG. 5 and Table 6 showed that most of the fragrance substances produced by the *Kluyveromyces marxianus* AMCC31343 were alcohols and esters at a flour paste fermentation phase, mainly including phenethanol, ethyl acetate and acetoin, among which phenethanol presented an fragrance of rose, ethyl acetate presented a fruity fragrance, and acetoin presented a butter fragrance. As the fermentation time increases, the concentrations of the main fragrance substances also changed differently. At the beginning of fermented flour paste (24 h), the content of each fragrance substance was relatively low. As the fermentation time increased, the content of a phenethanol substance in the AMCC31343 gradually increased at the fermentation metaphase (48 h), while the acetoin increased to the highest concentration. At the end of fermentation (60 h), the fragrance substances in the fermented flour paste of the commercially available strain began to decrease, while the content of phenethanol in AMCC31343 still increased, which was increased by 38% than that of the commercially available strain, the content of ethyl acetate in AMCC31343 was increased by 374% than that of the commercially available strain, and the content of acetoin in AMCC31343 was increased by 1535% than that of the commercially available strain.

The analysis results of the fragrance substances in fermented steamed buns in FIG. 6 and Table 7 showed that the concentrations of acetoin in the commercially available strain were relatively low and continued to decrease in the steamed buns made at different fermentation periods, while the content of acetoin in the *Kluyveromyces marxianusAMCC31343* continued to increase. The *Kluyveromyces marxianusAMCC31343* could produce 2,3-butanedione with a cream fragrance at different fermentation periods, while this fragrance substance was not detected in the commercially available strain. In the steamed bun made after 60 h of fermentation, the content of this fragrance substance in the *Kluyveromyces marxianus* AMCC31343 was 107 times higher than that in the commercially available strain. Therefore, a fragrance powder made from the *Kluyveromyces marxianus* AMCC31343 and other related products had a fragrance advantage over long fermentation, and can endow the product with a butter fragrance, fragrance of rose and fruity fragrance.

The foregoing descriptions are merely preferred examples of the present invention, and are not intended to limit the present invention in any form. Any modifications, equivalent substitutions, improvements, etc. made within the spirit and principles of the present invention should fall within the protection scope of the present invention.

## Claims

1. A *Kluyveromyces marxianus* strain, **characterized in that** the *Kluyveromyces marxianus* strain is *Kluyveromyces marxianus* AMCC30648, which is deposited in the China Center for Type Culture Collection (CCTCC), and the deposit number is CCTCC NO: M 20222108; or
*Kluyveromyces marxianus* AMCC31343, which is deposited in the China Center for Type Culture Collection (CCTCC), and the deposit number is CCTCC NO: M 20222111.

2. The strain according to claim 1, **characterized in that** an ITS gene sequence of the *Kluyveromyces marxianus* AMCC30648 is as shown in SEQ ID NO: 1.

3. The strain according to claim 1, **characterized in that** an ITS gene sequence of the *Kluyveromyces marxianus* AMCC31343 is as shown in SEQ ID NO: 2.

4. A fermented preparation method of a *Kluyveromyces marxianus* microbial agent, **characterized in that** the method comprises the following step: culturing the *Kluyveromyces marxianus* strain according to any one of claims 1 to 3.

5. The preparation method according to claim 4, the preparation method comprises the following steps:

(1) performing amplified culture on the *Kluyveromyces marxianus* strain according to any one of claims 1 to 3; and
(2) adding the product obtained in step (1) to a medium and performing fermented culture at 26-42°C;

preferably, the medium being a liquid medium or a solid medium.

6. A microbial agent, **characterized in that** the microbial agent contains the *Kluyveromyces marxianus* strain according to any one of claims 1 to 3.

7. The microbial agent according to claim 6, the microbial agent being obtained by the fermented preparation method according to claim 4 or 5.

8. A use of the *Kluyveromyces marxianus* strain according to any one of claims 1 to 3 or the microbial agent according to claim 6 or 7 in the fermentation.

9. A use of the *Kluyveromyces marxianus* strain according to any one of claims 1 to 3 or the microbial agent according to claim 6 or 7 in the fermentation of dairy products, fermented production of liquor or fermentation of flour products.

10. A flour dough, **characterized in that** the flour dough contains the *Kluyveromyces marxianus* strain according to any one of claims 1 to 3 or the microbial agent according to claim 6 or 7.

11. The flour dough according to claim 10, the flour dough contains one or a combination of two or more of acetoin, phenethanol, isoamyl acetate, phenethyl acetate, ethyl acetate, ethyl caprylate, and phenethyl isobutyrate.

12. A flour paste, the flour paste contains the *Kluyveromyces marxianus* strain according to any one of claims 1 to 3 or the microbial agent according to claim 6 or 7,

13. The flour dough according to claim 10, The flour paste according to claim 12, the flour paste contains one or a combination of two or more of acetoin, phenethanol, isoamyl acetate, phenethyl acetate, ethyl acetate, ethyl caprylate, and phenethyl isobutyrate.

14. The flour paste according to claim 12 or 13, **characterized in that** a mass ratio of the *Kluyveromyces marxianus* strain to flour to water in the flour paste is (0.1-5): (200-500): (500-1000).

15. A flour product, a fermentation microbial agent for the flour product contains the *Kluyveromyces marxianus* strain according to any one of claims 1 to 3 or the microbial agent according to claim 6 or 7.

16. The flour product according to claim 15, the flour product contains one or a combination of two or more of acetoin, phenethanol, and 2,3-butanedione.

17. A fermentation broth, a fermentation microbial agent for the fermentation broth contains the *Kluyveromyces marxianus* strain according to any one of claims 1 to 3 or the microbial agent according to claim 6 or 7.

18. The fermentation broth according to claim 17, the fermentation broth contains phenethyl acetate, phenethanol, ethyl acetate, isoamyl acetate, farnesol, phenethyl isobutyrate, and ethyl caprylate.

19. A liquor, a fermentation microbial agent for the liquor contains the *Kluyveromyces marxianus* strain according to any one of claims 1 to 3 or the microbial agent according to claim 6 or 7.

Fig. 1

Fig. 2

Fig. 3

Fig. 4

Fig. 5

Fig. 6

## INTERNATIONAL SEARCH REPORT

| International application No. |
| --- |
| **PCT/CN2024/100392** |

**A.    CLASSIFICATION OF SUBJECT MATTER**

C12N1/16(2006.01)i;  C12N1/02(2006.01)i;  A23C9/12(2006.01)i;  C12R1/645(2006.01)i

According to International Patent Classification (IPC) or to both national classification and IPC

**B.    FIELDS SEARCHED**

Minimum documentation searched (classification system followed by classification symbols)

   IPC:C12N,A23C,C12R.

Documentation searched other than minimum documentation to the extent that such documents are included in the fields searched

Electronic data base consulted during the international search (name of data base and, where practicable, search terms used)

   VEN, CNABS, PubMed, DWPI, CNTXT, WOTXT, USTXT, EPTXT, JPTXT, CNKI, ISI of Web Science, 百度学术, BAIDU SCHOLAR, BING, Patentics: 申请人, 发明人, 保藏, 马克斯克鲁维酵, 马克斯克鲁维酵母, 西藏, 乙偶姻, 乙酸苯乙酯, 乙酸乙酯, 乙酸乙酯 amd 乙偶姻, 乙酸异戊酯, 诱变, acetoin, AMCC30648, AMCC31343, CCTCC, Chymosin, xizang, Kluyverom yces marxianus, Kluyveromyces lactis , Ethyl acetate , acetoin, phenylethyl alcohol.

**C.    DOCUMENTS CONSIDERED TO BE RELEVANT**

| Category* | Citation of document, with indication, where appropriate, of the relevant passages | Relevant to claim No. |
| --- | --- | --- |
| A | CN 115895920 A (CHINA NATIONAL RESEARCH INSTITUTE OF FOOD & FERMENTATION INDUSTRIES CO., LTD. et al.) 04 April 2023 (2023-04-04) abstract, claims 1-3, and description, paragraphs 5-10 and 20 | 1-19 |
| A | CN 114032187 A (JALA (GROUP) CO., LTD.) 11 February 2022 (2022-02-11) claims 1-9 | 1-19 |
| A | WO 2020217150 A1 (KING FAISAL UNIVERSITY) 29 October 2020 (2020-10-29) claims 1-20 | 1-19 |
| A | CN 103333811 A (WUHAN POLYTECHNIC UNIVERSITY) 02 October 2013 (2013-10-02) claims 1-10 | 1-19 |
| A | CN 105087403 A (FUDAN UNIVERSITY) 25 November 2015 (2015-11-25) claims 1-10 | 1-19 |
| A | CN 107164246 A (KUNMING UNIVERSITY OF SCIENCE AND TECHNOLOGY) 15 September 2017 (2017-09-15) claims 1-5 | 1-19 |

☑ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **24 September 2024** | **27 September 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/100392** |

**C.    DOCUMENTS CONSIDERED TO BE RELEVANT**

| Category* | Citation of document, with indication, where appropriate, of the relevant passages | Relevant to claim No. |
| --- | --- | --- |
| A | 陈娟等 (CHEN, Juan et al.). "川西高原牧区传统发酵牦牛酸奶挥发性风味成分的分析 (Analysis of Volatile Components of Traditional Fermented Yak Yogurt in the Western Sichuan Plateau)" 食品工业科技 (*Science and Technology of Food Industry*), Vol. 37, No. 14, 31 December 2016 (2016-12-31), pages 53-56 | 1-19 |
| A | PU, Shunchang et al. "Yeasts from Chinese Strong Flavour Daqu Samples: Isolation and Evaluation of Their Potential for Fortified Daqu Production" *AMB Express*, Vol. 11, No. 176, 31 December 2021 (2021-12-31), pages 1-12 | 1-19 |

## INTERNATIONAL SEARCH REPORT

| International application No. |
| --- |
| **PCT/CN2024/100392** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☑ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2024/100392**

| Patent document cited in search report | | | Publication date (day/month/year) | Patent family member(s) | | | Publication date (day/month/year) |
|---|---|---|---|---|---|---|---|
| CN | 115895920 | A | 04 April 2023 | None | | | |
| CN | 114032187 | A | 11 February 2022 | None | | | |
| WO | 2020217150 | A1 | 29 October 2020 | US | 2021155960 | A1 | 27 May 2021 |
| | | | | US | 2020340018 | A1 | 29 October 2020 |
| | | | | US | 10870868 | B2 | 22 December 2020 |
| CN | 103333811 | A | 02 October 2013 | None | | | |
| CN | 105087403 | A | 25 November 2015 | None | | | |
| CN | 107164246 | A | 15 September 2017 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS*, 103-45-7
- *CHEMICAL ABSTRACTS*, 60-12-8
- *CHEMICAL ABSTRACTS*, 141-78-6
- *CHEMICAL ABSTRACTS*, 123-92-2
- *CHEMICAL ABSTRACTS*, 4602-84-0
- *CHEMICAL ABSTRACTS*, 103-48-0
- *CHEMICAL ABSTRACTS*, 106-32-1
- *CHEMICAL ABSTRACTS*, 513-86-0
- *CHEMICAL ABSTRACTS*, 431-03-8